# EUROPÄISCHE PATENTSCHRIFT

(11) **EP 1 185 279 B1**
(45) Veröffentlichungstag und Bekanntmachung des Hinweises auf die Patenterteilung: **26.01.2005**
(21) Anmeldenummer: 00949114.3
(22) Anmeldetag: 20.06.2000
(51) Int. Cl.: A61K 35/76, A61K 48/00, A61P 35/00, A61K 38/17

(54) **MITTEL ZUR BEHANDLUNG MALIGNER ERKRANKUNGEN UNTER VERWENDUNG DES PROTEINS YB-1**
AGENTS FOR TREATING MALIGNANT DISEASES USING E1A-DEFICIENT ADENOVIRUSES WITH YB-1 PROTEIN-DEPENDENT REPLICATION
AGENTS POUR TRAITER DES AFFECTIONS MALIGNES AU MOYEN D'ADENOVIRUS DEFICIENTS E1A ET DEPENDANTS DE LA PROTEINE YB-1 POUR LEUR REPLICATION

(30) Priorität: 21.06.1999 DE 19929569
(43) Veröffentlichungstag der Anmeldung: 13.03.2002
(73) Patentinhaber: Holm, Per Sonne, 81675 München (DE)
(72) Erfinder: HOLM, Per, Sonne, D-81675 München (DE); ROYER, Hans-Dieter, D-40225 Düsseldorf (DE); DIETEL, Manfred, D-14193 Berlin (DE); LAGE, Hermann, D-13353 Berlin (DE); LADHOFF, Axel, D-16562 Bergfelde (DE); JÜRCHOTT, Karsten, D-15345 Altlandsberg (DE); BERGMANN, Stephan, D-10245 Berlin (DE); BRAND, Karsten, D-10555 Berlin (DE)
(74) Vertreter: Bohmann, Armin K., Dr.
(86) Internationale Anmeldenummer: PCT/DE2000/001978
(87) Internationale Veröffentlichungsnummer: WO 2000/078327

(56) Entgegenhaltungen:
- WO-A-94/18992
- US-A- 5 856 181
- KIRN D (REPRINT) ET AL: "Adenovirus E1A mutants that selectively replicate in and cause enhanced destruction of cancer cells in vitro and in nude mouse-human tumor xenografts" CANCER GENE THERAPY, (NOV-DEC 1998) VOL. 5, NO. 6, SUPP. [S], PP. O79-O79. PUBLISHER: APPLETON & LANGE, 25 VAN ZANT ST, E NORWALK, CT 06855. ISSN: 0929-1903., - 21. November 1998 (1998-11-21) XP000982296 ONYX PHARMACEUT, RICHMOND, CA
- BARGOU RALF C ET AL: "Nuclear localization and increased levels of transcription factor YB-1 in primary human breast cancers are associated with intrinsic MDR1 gene expression." NATURE MEDICINE, Bd. 3, Nr. 4, 1997, Seiten 447-450, XP002160622 ISSN: 1078-8956 in der Anmeldung erwähnt
- HEISE ET AL: "ONYX-015, an E+B gene-attenuated adenovirus, causes tumor-specific cytolysis and antitumoral efficacy that can be augmented by standard chemotherapeutic agents" NATURE MEDICINE,US,NATURE PUBLISHING, CO, Bd. 3, Nr. 6, 1. Juni 1997 (1997-06-01), Seiten 639-645, XP002095383 ISSN: 1078-8956

## Beschreibung

Die Erfindung betrifft Mittel zur Behandlung maligner Erkrankungen. Mit Hilfe der erfinderischen Lösung wird es möglich, eine E1A-unabhängige Replikation von Adenoviren in Tumorzellen zu bewirken, um damit diese Tumorzellen zu zerstören. Außerdem wird es ermöglicht, durch den Einsatz von E1A-defekten Adenoviren Tumorzellen zu zerstören, die das Protein YB-1 im Kern aufweisen. Anwendungsgebiete sind die Medizin und die pharmazeutische Industrie.

Bei dem Protein YB-1 handelt es sich um einen Vertreter der Y-Box-Proteinfamilie, die an das DNA-Sequenzmotiv Y-Box bindet. Das Y-Box-Motiv stellt ein transkriptionell regulatorisches Element dar, das sich in den Promoter- oder Enhancer-Regionen einer Anzahl unterschiedlicher Gene findet, die eine Rolle bei der Regulation der Zellproliferation spielen (Ladomery, M. et al., 1995, Bioassays 17:9-11; Didier, D. K. et al., 1988, PNAS, 85, 7322-7326).
Ober die Rolle des Proteins E1A bei der Adenovirus-Replikation berichten Flint et al. (Flint, J. and Shenk, T. A., 1989, Rev. Genet., 23, 141-161).
Den Zusammenhang von YB-1 (Faktor YB-1) und MDR-1-Gen-Expression (MDR - multidrug resistance) sowie der Lokalisation von YB-1 im Zellkem und P-Glycoprotein (Pgp oder P-170) haben Bargou et al. untersucht (Bargou, R. C. et al., Nature Med. 3, 1997, 4:447-450). Bei der Analyse von Mammakaninomgewebe wurde eine deutliche Überexpression von YB-1 im Vergleich zum normalen Brustepithel nachgewiesen. Zudem wurde gezeigt, daß es nach der Lokalisation des Faktors YB-1 in den Kern zur Synthese des P-Glycoproteins kommt und damit die Ausbildung des Multidrug-Resistenz Phänotyps bewirkt wird. Von allen untersuchten Tumoren wiesen 30 % YB-1 zusätzlich im Kern auf. Von diesen exprimierten alle (100 %) das P-Glycoprotein.

US-Patentanmeldung 5,856,181 betrifft die Verwendung von E1A-defizienten Adenoviren zur Behandlung solcher Tumoren, die eine gestörte p53- und/oder RB-Funktion aufweisen.

Kirn D. (Reprint) et al., Cancer Gene Therapy, (Nov.-Dez. 1998), Bd. 5, Nr. 6, Supp [S], Seiten O69-O79 offenbaren die Verwendung von Adenviren zur Onkolyse, die Mutationen in der pRB-Bindungsregion (CR-1) oder in der p300-Bindungsregion (CR-2) aufweisen.

Heise et al., Nature Medicine, US, Nature publishing, CO, Bd. 3, Nr. 1, 1. Juni 1997, Seiten 639-647 beschreiben die Onkolyse durch EIB-Gen-attenuierte Adenoviren, die eine Replikation in Abhängigkeit von p53 zeigen.

Der Erfindung liegt die Aufgabe zugrunde, neue Mittel zur Verfügung zu stellen, die es erlauben, neoplastisches Gewebe zu zerstören. Die Aufgabe wurde erfindungsgemäß dadurch gelöst, daß Adenoviren mit YB-1-abhängiger Replikationskompetenz oder E1A-defiziente Adenoviren eingesetzt werden.

Die Aufgabe wird erfindungsgemäß gelöst durch den Gegenstand der unabhängigen Ansprüche, wobei sich bevorzugte Ausführungsformen aus den Unteransprüchen ergeben.

Die Erfinder konnten zeigen, daß E1A-defiziente (E1A-defekte) Adenoviren überraschenderweise in Zellen zur Replikation fähig sind, die den Faktor YB-1 im Kern enthalten. Die praktische Vorgehensweise besteht darin, YB-1-exprimierende Tumorzellen mit E1A-defizienten Adenoviren zu infizieren. Da das Virus nur in Zellen repliziert, in denen sich YB-1 im Kern befindet, werden auch nur diese Zellen durch die Virusvermehrung zerstört.
Mit dieser Erfindung werden Mittel zur E1A-unabhängigen Replikation von rekombinanten, die YB-1 kodierende DNA-Sequenz tragende Adenoviren beansprucht, die Zellen zur Synthese des rekombinanten, adenoviralen YB-1 veranlassen. Damit betrifft die Erfindung die Verwendung von YB-1 zur E1A-unabhängigen Replikation von Adenoviren. Das Wesen der Erfindung besteht darin, daß ein replikativ defektes E1A-Adenovirus durch die Synthese von YB-1 wieder in Zellen replizierbar wird und dadurch die Tumorzelle zerstört.
Beansprucht werden Mittel zur Behandlung maligner Erkrankungen, basierend auf dem Einsatz von E1A-defizienten Adenoviren mit YB-1 abhängiger Replikation sowie des weiteren Mittel zur E1A-unabhängigen Replikation von Adenoviren, gekennzeichnet dadurch, daß sie YB-1-vermittelt sind. Diese Mittel können weitere tumorzeilschädigende Substanzen enthalten.
Ein weiteres Merkmal der Erfindung besteht in der Verwendung des Proteins YB-1 zur E1Aunabhängigen Replikation von Adenoviren. Es gehören ferner zu den Merkmalen der Erfindung: Die Verwendung von E1A-defekten Adenoviren zur Zerstörung von Tumorzellen, die im Kern das Protein YB-1 enthalten sowie die Verwendung von rekombinanten, die YB-1 kodierende DNA-Sequenz tragenden E1A-defekten Adenoviren in der Therapie und in der biomedizinischen Forschung.

Die Merkmale der Erfindung gehen außer aus den Ansprüchen auch aus der Beschreibung hervor, wobei die einzelnen Merkmale jeweils für sich allein oder zu mehreren in Form von Kombinationen vorteilhafte schutzfähige Ausführungen darstellen, für die mit dieser Schrift Schutz beantragt wird. Die Kombination besteht aus bekannten (Protein YB-1, E1Adefizienten Adenoviren) und neuen Elementen (Infizieren von Zellen mit E1A-defizienten Adenoviren), die sich gegenseitig beeinflussen und in ihrer neuen Gesamtwirkung einen Vorteil (synergistischen Effekt) und den erstrebten Erfolg ergeben, der darin liegt, daß nunmehr Tumorzellen zerstört und gesunde Zellen nicht beeinträchtigt werden.
Ein weiteres Kombinationsmerkmal besteht darin, daß die erfindungsgemäßen Mittel gemeinsam mit anderen tumorzellschädigenden Substanzen - z.B. Zytostatika oder Ribozymen - oder anderen therapeutischen Ansätzen - wie chirurgische Tumorexzision, Strahlentherapie, Chemotherapie, Hyperthermie oder Gentherapie - Verwendung finden.

Die Erfindung soll anhand von Ausführungsbeispielen näher erläutert werden, ohne auf diese Beispiele beschränkt zu sein.

### Ausführungsbeispiele

### Beispiel 1

Klinischer Einsatz von ElA-defekten Adenoviren gegen neoplastische Erkrankungen, auch in Kombination mit anderen therapeutischen Ansätzen, wie z.B. chirurgische Tumorexzision, Strahlentherapie, Chemotherapie, Einsatz von Hyperthermie, Gentherapie.

### Beispiel 2

Einsatz in der biomedizinischen Forschung zur Entwicklung von verbesserten gentherapeutischen Verfahren.

## Patentansprüche

1. Verwendung von E1A-defizienten Adenoviren zur Herstellung eines Medikamentes zur Behandlung von YB-1 exprimierenden Tumoren.

2. Verwendung nach Anspruch 1, **dadurch gekennzeichnet, dass** die Tumoren YB-1 im Kern enthalten.

3. Verwendung nach Anspruch 1 oder 2, **dadurch gekennzeichnet, dass** weitere tumorzellschädigende Substanzen mit den E1A-defizienten Adenoviren verwendet werden.

4. E1A-defizienter Adenovirus umfassend eine YB-1 kodierende DNA-Sequenz.

5. Verwendung von ElA-defizienten Adenoviren nach Anspruch 4 zur Herstellung eines Medikamentes.

6. In vitro Verwendung von YB-1 zur E1A-unabhängigen Replikation von Adenoviren.

7. Verwendung von YB-1 zur Herstellung eines Medikamentes zur E1A-unabhängigen Replikation von Adenoviren in Tumoren.

## Claims

1. Use of E1A deficient adenoviruses for the manufacture of a medicament for the treatment of YB-1 expressing tumors.

2. Use according to claim 1, **characterised in that** the tumors have YB-1 in the nucleus.

3. Use according to claim 1 or 2, **characterised in that** further tumor cell damaging substances are used with the E1A deficient adenoviruses.

4. E1A deficient adenovirus comprising a YB-1 coding DNA sequence.

5. Use of E1A deficient adenoviruses according to claim 4 for the manufacture of a medicament.

6. In vitro use of YB-1 for E1A independent replication of adenoviruses.

7. Use of YB-1 for the manufacture of a medicament for E1A independent replication of adenoviruses in tumors.

## Revendications

1. Utilisation d'adénovirus déficients E1A pour la préparation d'un médicament pour le traitement de tumeurs exprimant la protéine YB-1.

2. Utilisation selon la revendication 1, **caractérisée en ce que** les tumeurs expriment la protéine YB-1 dans leur noyau.

3. Utilisation selon la revendication 1 ou 2, **caractérisée en ce que** d'autres substances portant atteinte aux cellules tumorales sont utilisées avec les adénovirus déficients E1A.

4. Adénovirus déficients E1A comprenant une séquence ADN codant pour la protéine YB-1.

5. Utilisation d'adénovirus déficients E1A selon la revendication 4 pour la préparation d'un médicament.

6. Utilisation *in vitro* de la protéine YB-1 pour la réplication indépendante E1A d'adénovirus.

7. Utilisation de la protéine YB-1 pour la préparation d'un médicament destiné à la réplication indépendante E1A d'adénovirus dans des tumeurs.
